# EUROPEAN PATENT SPECIFICATION

(11) **EP 2 785 672 B1**
(45) Date of publication and mention of the grant of the patent: **26.10.2016**
(21) Application number: 12766786.3
(22) Date of filing: 13.09.2012
(51) Int. Cl.: C07C 29/80, C07C 29/88, C07C 33/046

(54) **CAUSTIC TREATMENT OF FORMALDEHYDE RECYCLE COLUMN FEED**
ÄTZBEHANDLUNG DES ZUSTROMS EINER FORMALDEHYD-RECYCLE-SÄULE
TRAITEMENT CAUSTIQUE D'UNE ALIMENTATION DE COLONNE DE RECYCLAGE DE FORMALDÉHYDE

(30) Priority: 28.11.2011 US 201161563875 P
(43) Date of publication of application: 08.10.2014
(73) Proprietor: Invista Technologies S.à.r.l., 9000 St. Gallen (CH)
(72) Inventor: BADAT, Hashim, M., Houston, TX 77062-2103 (US); GAUSE, Jason, C., Baytown, TX 77523-8550 (US)
(74) Representative: Carpmaels & Ransford LLP
(86) International application number: PCT/US2012/055149
(87) International publication number: WO 2013/081713

(56) References cited:
- EP-A1- 0 309 915
- US-A- 2 993 078
- US-A- 4 180 687
- US-A- 4 319 055

## Description

### FIELD OF THE INVENTION

This disclosure relates to a process for producing 1,4 butynediol. More specifically, it relates the improved efficiency during the distillation recovery of excess formaldehyde from crude 1,4 butynediol solutions.

### BACKGROUND OF THE INVENTION

1,4-Butynediol (BYD) is a commonly produced organic compound. BYD is a useful intermediate chemical in the production of pesticides, textile additives, corrosion inhibitors, plasticizers, synthetic resins, butanediol, tetrahydrofuran, polyether polyols and polyurethanes.

It is well known that BYD can be produced by the Reppe process, via the reaction of acetylene and formaldehyde. Several patents, such as U.S. Patent Nos. 2,300,969, 3,560,576, 4,093,668 and 4,127,734, teach the production of BYD through Reppe chemistry.

Many commercial processes for the synthesis of BYD operate with excess formaldehyde, thereby improving yields. In the process of manufacturing BYD, an aqueous solution, which is referred to as crude BYD, may contain 30 to 40% BYD and 3 to 12% formaldehyde. Once a crude BYD stream has been produced, it is desirable to remove unreacted formaldehyde from the stream to retrieve a purified BYD product stream that can be further processed. Generally, the crude BYD can be distilled to remove formaldehyde which is recycled in the system. The distillation step is expensive both in the initial cost of equipment and in energy consumption during use.

Several other known process also exist for removing excess formaldehyde from a BYD stream. For example, European Pat No. 309915 teaches a process wherein excess formaldehyde is removed from aqueous solutions of BYD by adding methanol and an acidic agent to the aqueous BYD solution and distilling off the dimethyl formal from the mixture at elevated temperatures. U.S. Patent No. 5,973,213 teaches the separation of solids from aqueous BYD solutions by passing a solids-containing aqueous BYD solution in the downflow mode through a column and thus bringing it into contact with a solvent which has a lower density than the solids-containing BYD solution and forms a second phase with the latter, with the solvent rising in countercurrent to the aqueous BYD solution, the solid accumulating at the interface between the aqueous BYD and the solvent and the solid being removed from the column by taking off a mixture of aqueous BYD and solvent.

U.S. Patent No. 7,605,292 relates to a process that comprises compressing BYD to from 50 to 1500 bar, depressurizing it, waiting for phase separation to occur after depressurization and separating off the bottom phase. U.S. Patent No. 4,180,687 teaches the removal of formaldehyde from crude BYD, wherein, the formaldehyde can be reacted to a polymeric substance in the presence of NaOH or Na.sub.2 CO.sub.3 at high temperatures. U.S. Patent No. 4,319,055 relates to a process for removing formaldehyde from aqueous solutions of BYD by treating the solutions with an alkaline agent at an elevated temperature in the presence of hydrogen peroxide. U.S. Patent No. 2.993,078 teaches an alternative to fractional distillation by purifying BYD via a treatment with an alkaline substance. All of these processes involve costly intermediate steps and additional processing equipment in order to remove excess formaldehyde from the crude BYD stream. Therefore, it is desirable to improve the existing distillation process for purifying BYD, so that the column efficiency is increased and the energy required for the process is decreased.

It is readily apparent from the physical properties of the major components of crude BYD that distillation would lend itself to separating the excess CH₂O from the crude BYD for recycle to the first reaction step. In a formaldehyde recycle distillation column, the formaldehyde is removed so that formaldehyde and some of the water is distilled overhead and recycled directly or indirectly back to the first reaction step. The bottoms from the distillation column, is comprised of a concentrated BYD stream and is fed forward for further processing.

It has been found that under typical operating conditions, the column operates at a lower effective capacity than would be predicted based upon the previously understood behavior of the components to be separated. Additionally, the column requires a higher steam duty than would normally be expected. It has not been fully understood why the operation appears to operate inefficiently.

Therefore, there is a need for a method of improving the recycle distillation column efficiency and decreasing the energy usage in a process for removing formaldehyde from a crude BYD stream.

### SUMMARY OF THE INVENTION

The present invention relates to a process for improving the recycle distillation column efficiency and decreasing the energy usage in a process for removing formaldehyde from a crude 1,4 butynediol (BYD) stream by controlling the pH level of the crude BYD stream prior to removing the formaldehyde.

The crude BYD stream is produced from a process of forming BYD through the reaction of formaldehyde and acetylene. An embodiment of the present invention comprises the steps of:
(a) providing a crude butynediol stream containing butynediol and formaldehyde;
(b) increasing the pH of the crude butynediol stream and forming a treated product stream;
(c) flowing said treated product stream into the inlet of a continuous distillation column;
(d) recovering a concentrated formaldehyde stream from the overhead stream of the distillation column; and
(e) recovering a concentrated butynediol stream from the bottoms stream of the distillation column.

In another embodiment, the pH of the treated product stream is raised to a level that limits the reaction of butynediol and formaldehyde to form acetal complex and decreases the solubility of trace metals found in the treated product stream, wherein the trace metals precipitate in the treated product stream.

In another embodiment, the pH level of the treated product stream is increased to a range of 5.5 to 10.

In another embodiment, the pH level of the treated product stream is increased through the addition of a pH control agent.

In another embodiment, the pH control agent is an aqueous sodium hydroxide solution.

In another embodiment, the pH of the treated product stream is increased though the use of ion exchange resins.

In another embodiment, the concentration of formaldehyde in the distillation bottoms stream is 1.0% by weight or less.

In another embodiment, the concentration of formaldehyde in the distillation bottoms stream is from 0.1 to 1.0 % by weight.

In another embodiment, the process further comprises the step of flowing said treated product stream through a filtration system to remove trace metals prior to step (c).

Another embodiment of the present invention discloses a method for reducing the steam consumption needed for a process for removing formaldehyde from a crude butynediol product stream comprising the steps of:
(a) providing a crude butynediol stream containing butynediol and formaldehyde;
(b) increasing the pH of the crude butynediol stream and forming a treated product stream;
(c) flowing said treated product stream into the inlet of a continuous distillation column;
(d) recovering a concentrated formaldehyde stream from the overhead stream of the distillation column; and
(e) recovering a concentrated butynediol stream the bottoms stream of the distillation column.

In another embodiment, the pH of the treated product stream is raised to a level that limits the reaction of butynediol and formaldehyde to form acetal complex and decreases the solubility of trace metals found in the treated product stream, wherein the trace metals precipitate in the treated product stream.

In another embodiment, the pH level of the treated product stream is increased to a range of 5.5 to 10.

In another embodiment, the pH level of the treated product stream is increased through the addition of a pH control agent.

In another embodiment, the pH control agent is an aqueous sodium hydroxide solution.

In another embodiment, the pH of the treated product stream is increased though the use of ion exchange resins.

In another embodiment, the concentration of formaldehyde in the distillation bottoms stream is 1.0% by weight or less.

In another embodiment, the concentration of formaldehyde in the distillation bottoms stream is from 0.1 to 1.0 % by weight.

In another embodiment, the method further comprises the step of flowing said treated product stream through a filtration system to remove trace metals prior to step (c).

### BRIEF DESCRIPTION OF THE DRAWINGS

FIG. 1 is a process diagram for an embodiment of the present invention.
FIG. 2 is a chart showing the steam consumption of the recycle distillation column as the pH on the column inlet stream is increased.

### DETAILED DESCRIPTION OF THE INVENTION

The present invention relates to a process for improving the recycle distillation column efficiency and decreasing the energy usage in a process for removing formaldehyde from a crude 1,4 butynediol (BYD) stream by controlling the pH level of the crude BYD stream prior to removing the formaldehyde.

The term "BYD", as used herein, unless otherwise indicated, refers to 1,4 butynediol. The general formula for BYD is HOCH₂CCCH₂OH. BYD is also known as butynediol, but-2-yne-1,4,diol, 2-Butyne-1,4-diol and 1,4-dihydroxy-2-butyne.

The term "acetal complex", as used herein, unless otherwise indicated, refers to acetal and hemiacetal compounds that are formed from the reaction of BYD and formaldehyde in the distillation column. The general formula for an acetal is R¹HC(OR)²OR³, where R¹, R², or R³ are often hydrogen. The general formula for a hemiacetal is R¹R²C(OH)OR, where R¹ or R² is often hydrogen.

The term "caustic addition", as used herein, unless otherwise indicates, refers to a strong basic solution. In specific embodiments of the present invention, sodium hydroxide (NaOH) may be utilized as a caustic addition.

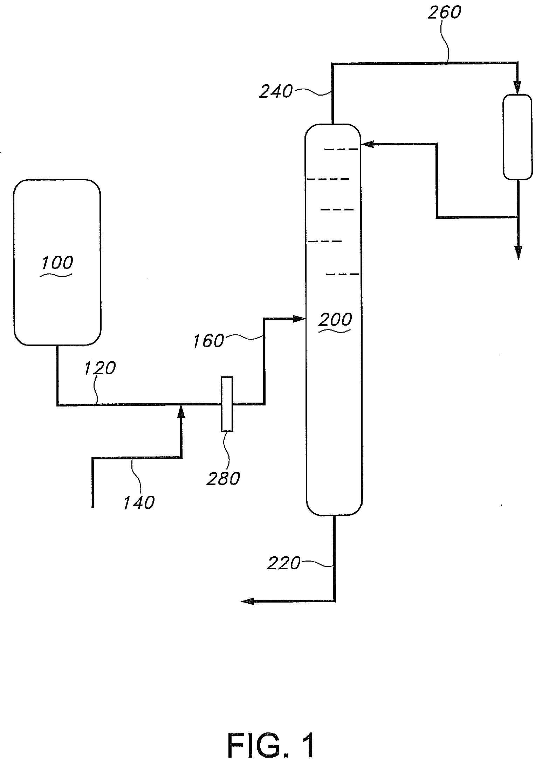

Referring to FIG. 1, an exemplary embodiment of the present invention is herein described. Crude BYD is produced via the reaction of acetylene and formaldehyde (CH₂O) (100). It is desirable to remove excess formaldehyde from the crude BYD stream (120), before the BYD is further processed. This can be accomplished in recycle distillation column (200), wherein a purified BYD product stream (220), is recovered from the recycle distillation column bottoms and the excess formaldehyde (240, 260) is recovered from the top of the column and can be recycled to further produce BYD.

In normal operation, the BYD would go down the column because of its relatively high boiling point, along with some water. CH₂O would tend to go up the column, with some water. In one embodiment, the CH₂O ranges from about 5 to 12% at the feed point, down to 0.1 to 1.0% in the bottoms from the column (220). The concentration of CH₂O going up the column ranges from 5 to 12% at the feed point, to 20 to 30% in the overheads (240,260).

The applicants have discovered that a caustic addition (140) to the crude BYD stream (120) to form a treated product stream (160) to the feed to the recycle distillation column (200) improves the efficiency in separating the CH₂O and water overhead (240, 260) for recycle and significantly reduces steam use by up to 25% of the steam that would otherwise be consumed. The underlying mechanism is not fully understood, and while not to limit the disclosed process by a recitation of theory, it is believed that increasing the pH in accordance with the disclosed process inhibits the formation of constituents that otherwise interfere with efficient operation of the recycle distillation column (200). One theory is that the reduction in steam use arises because of a reduction in acetal complex, which comprises of acetals and hemiacetals made from the reaction of BYD and CH₂O. The caustic addition (140) lowers the pH of the crude BYD stream (120) by neutralizing the organic acids in the stream to form organic salts and water. For example, formic acid that maybe present in the crude BYD stream (120) will be neutralized to sodium formate by the addition of a (caustic) sodium hydroxide addition.

CH₂O in aqueous solutions reacts with water in an equilibrium fashion to make methanol (MeOH) and formic acid HCOOH) as by-products as a function of time. Given time, the pH of the crude BYD will increase in HCOOH content thereby reducing the pH of the crude BYD to between 4 and 4.5. Caustic is added to raise the pH to a range of about 5.5 to about 10 prior to feeding the crude BYD to the CH₂O recycle column. In an embodiment of the present invention, the caustic treatment may be an aqueous sodium hydroxide solution.

In other embodiments of the current invention, the pH of the crude BYD stream (120) maybe increased through other methods known in the art. In a particular embodiment of the current invention, the pH of the crude BYD stream (120) can be increased by the removal of organic acids through the use of ion exchange resins. An example of ion exchange resins that may be used are anion exchange resins such as AMBERLITET^{™} IRA96 or DOWEX^{™} M-43 that act as acid absorbers to neutralize the crude BYD stream(120). Other known methods of removing organic acids from process stream may also be used. For example, an adsorbent such as activated carbon may also be used to neutralize the crude BYD stream(120).

The applicants have also found that increasing the pH of the treated product stream (160) significantly decreases the solubility of trace metals, such as copper, that are found in the crude BYD stream (120). Once the solubility of the trace metals has been decreased, the precipitated trace metals may then be removed from the treated product stream though a filtration system (280) prior to entering the recycle distillation column (200). Any filtration system known in the art, such as a cartridge filter system, may be used to remove the trace metals from the treated product stream (160). The removal of these trace metals is beneficial because they can contribute to fouling in the recycle distillation column (200) and also contribute to catalyst poisoning downstream when the purified BYD product stream (220) is further processed.

### Examples

The following Examples demonstrate the present invention and its capability for use. The invention is capable of other and different embodiments, and its several details are capable of modifications in various apparent respects, without departing from the scope and spirit of the present invention. Accordingly, the Examples are to be regarded as illustrative in nature and non-limiting.

### Example 1

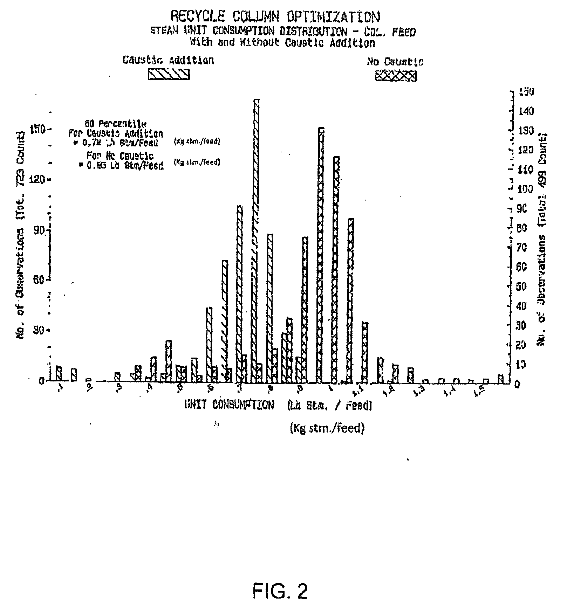

FIG.2 is a chart showing the observed steam usage at the INVISTA LaPorte site with and without a caustic addition to the recycle distillation column feed. The recycle distillation column (200) was operated at a pressure range between 3.79-4.48 bar (55-65 psig). The temperature at the feed point (160) was between 50-70°C, the bottoms temperature (220) was between 155-177°C and the temperature at the top of the column (240) was between 140-150°C. The reflux temperature was between 80-120°C and the reflux ratio was maintained at 0.5-0.8 (reflux to feed). The chart in FIG. 2 shows the steam consumption data for a recycle distillation column. The pounds of steam used per pound of crude BYD feed Steam were tracked. The data was taken for period when the pH of the BYD inlet stream was unregulated and for a period when the pH was increased by the addition of aqueous sodium hydroxide (25-50% wt NaOH). Prior to the caustic addition, the average pH of the BYD inlet stream was observed to be about 5.35. Over an 18 week period, the pH of the BYD inlet stream was increased and regulated at a range of 5.7-6.4 through the caustic addition. The results of the study are summarized in FIG. 2. The amount of steam used for the column was reduced from 0.95 kg steam/kg feed to 0.72 kg steam/kg feed (0.95 Ib steam/lb feed to 0.72 lb steam/Ib feed). This indicates that the efficiency of the column was improved by raising the pH level of the BYD inlet stream to the recycle distillation column.

### Example 2

An improved method for removing formaldehyde from a crude butynediol product stream comprising the step of providing a crude butynediol stream containing butynediol and formaldehyde. The pH of the crude butynediol stream is then increased to form a treated product stream. The treated product stream then flows into the inlet of a continuous distillation column. Finally, a concentrated formaldehyde stream from the overhead stream of the distillation column and a concentrated butynediol stream from the bottoms stream of the distillation column are both recovered.

### Example 3

The process of Example 2 is repeated with additional steps. In this example, the pH of the treated product stream is raised to a level that limits the reaction of butynediol and formaldehyde to form acetal complex and decreases the solubility of trace metals found in the treated product stream, wherein the trace metals precipitate in the treated product stream.

### Example 4

The process of Example 3 is repeated with additional steps. In this example, the pH level of the treated product stream is increased to a range of 5.5 to 10.

### Example 5

The process of Example 4 is repeated with additional steps. In this example, pH level of the treated product stream is increased through the addition of a pH control agent.

### Example 6

The process of Example 5 is repeated with additional steps. In this example, the pH control agent is an aqueous sodium hydroxide solution.

### Example 7

The process of Example 6 is repeated with additional steps. In this example, the pH of the treated product stream is increased though the use of ion exchange resins.

### Example 8

The process of Example 7 is repeated with additional steps. In this example, the concentration of formaldehyde in the distillation bottoms stream is 1.0% by weight or less.

### Example 9

The process of Example 8 is repeated with additional steps. In this example, the concentration of formaldehyde in the distillation bottoms stream is from 0.1 to 1.0 % by weight.

### Example 10

The process of Example 9 is repeated with additional steps. In this example, said treated product stream flows through a filtration system to remove trace metals prior to entering the continuous distillation column.

### Example 11

A method for reducing the steam consumption needed for removing formaldehyde from a crude butynediol product stream comprising the step of providing a crude butynediol stream containing butynediol and formaldehyde. The pH of the crude butynediol stream is then increased to form a treated product stream. The treated product stream then flows into the inlet of a continuous distillation column. Finally, a concentrated formaldehyde stream from the overhead stream of the distillation column and a concentrated butynediol stream from the bottoms stream of the distillation column are both recovered.

### Example 12

The process of Example 11 is repeated with additional steps. In this example, the pH of the treated product stream is raised to a level that limits the reaction of butynediol and formaldehyde to form acetal complex and decreases the solubility of trace metals found in the treated product stream, wherein the trace metals precipitate in the treated product stream.

### Example 13

The process of Example 12 is repeated with additional steps. In this example, the pH level of the treated product stream is increased to a range of 5.5 to 10.

### Example 14

The process of Example 13 is repeated with additional steps. In this example, pH level of the treated product stream is increased through the addition of a pH control agent.

### Example 15

The process of Example 14 is repeated with additional steps. In this example, the pH control agent is an aqueous sodium hydroxide solution.

### Example 16

The process of Example 15 is repeated with additional steps. In this example, the pH of the treated product stream is increased though the use of ion exchange resins.

### Example 17

The process of Example 16 is repeated with additional steps. In this example, the concentration of formaldehyde in the distillation bottoms stream is 1.0% by weight or less.

### Example 18

The process of Example 17 is repeated with additional steps. In this example, the concentration of formaldehyde in the distillation bottoms stream is from 0.1 to 1.0 % by weight.

### Example 19

The process of Example 18 is repeated with additional steps. In this example, said treated product stream flows through a filtration system to remove trace metals prior to entering the continuous distillation column.

## Claims

1. An improved method for removing formaldehyde from a crude butynediol product stream comprising the steps of:
(a) providing a crude butynediol stream containing butynediol and formaldehyde;
(b) increasing the pH of the crude butynediol stream and forming a treated product stream;
(c) flowing said treated product stream with an increased pH into the inlet of a continuous distillation column;
(d) recovering a concentrated formaldehyde stream from the overhead stream of the distillation column; and
(e) recovering a concentrated butynediol stream from the bottoms stream of the distillation column.

2. The method of claim 1 wherein of the pH of the treated product stream is raised to a level that limits the reaction of butynediol and formaldehyde to form acetal complex and decreasing the solubility of trace metals found in the treated product stream, wherein the trace metals precipitate in the treated product stream.

3. The method of claim 1 wherein the pH level of the treated product stream is increased to a range of 5.5 to 10.

4. The method of claim 1 wherein the pH level of the treated product stream is increased through the addition of a pH control agent.

5. The method of claim 4 wherein the pH control agent is an aqueous sodium hydroxide solution.

6. The method of claim 1 wherein the pH of the treated product stream is increased though the use of ion exchange resins.

7. The method of claim 1 wherein the concentration of formaldehyde in the distillation bottoms stream is 1.0% by weight or less.

8. The method of claim 7 wherein the concentration of formaldehyde in the distillation bottoms stream is from 0.1 to 1.0 % by weight.

9. The method of claim 1 further comprising the step of flowing said treated product stream through a filtration system to remove trace metals prior to step (c).

## Patentansprüche

1. Verbessertes Verfahren zur Entfernung von Formaldehyd aus einem Rohbutindiol-Produktstrom, das folgende Schritte umfasst:
(a) Bereitstellen eines Rohbutindiolstroms, der Butindiol und Formaldehyd enthält;
(b) Erhöhen des pH-Werts des Rohbutindiolstroms und Bilden eines behandelten Produktstroms;
(c) Einleiten des behandelten Produktstroms mit einem erhöhten pH-Wert in den Einlass einer kontinuierlich arbeitenden Destillationssäule;
(d) Gewinnen eines konzentrierten Formaldehydstroms aus dem Kopfstrom der Destillationssäule und
(e) Gewinnen eines konzentrierten Butindiolstroms aus dem Sumpfstrom der Destillationssäule.

2. Verfahren nach Anspruch 1, bei dem der pH-Wert des behandelten Produktstroms auf ein Niveau angehoben wird, bei dem die Reaktion von Butindiol und Formaldehyd unter Bildung eines Acetalkomplexes eingeschränkt und die Löslichkeit von in dem behandelten Produktstrom anzutreffenden Spurenmetallen herabgesetzt wird, wobei die Spurenmetalle in dem behandelten Produktstrom ausfallen.

3. Verfahren nach Anspruch 1, bei dem das pH-Niveau des behandelten Produktstroms auf einen Bereich von 5,5 bis 10 erhöht wird.

4. Verfahren nach Anspruch 1, bei dem das pH-Niveau des behandelten Produktstroms durch Zugabe eines den pH-Wert regulierenden Mittels erhöht wird.

5. Verfahren nach Anspruch 4, wobei es sich bei dem den pH-Wert regulierenden Mittel um eine wässrige Natriumhydroxidlösung handelt.

6. Verfahren nach Anspruch 1, bei dem der pH-Wert des behandelten Produktstroms durch die Verwendung von Ionenaustauscherharzen erhöht wird.

7. Verfahren nach Anspruch 1, bei dem die Formaldehydkonzentration im Destillationssumpfstrom 1,0 Gew.-% oder weniger beträgt.

8. Verfahren nach Anspruch 7, bei dem die Formaldehydkonzentration im Destillationssumpfstrom 0,1 bis 1,0 Gew.-% beträgt.

9. Verfahren nach Anspruch 1, das ferner den Schritt des Durchleitens des behandelten Produktstroms durch ein Filtrationssystem zur Entfernung von Spurenmetallen vor Schritt (c) umfasst.

## Revendications

1. Procédé amélioré pour éliminer le formaldéhyde d'un flux de produit de butynediol brut comprenant les étapes de :
(a) fourniture d'un flux de butynediol brut contenant du butynediol et du formaldéhyde ;
(b) augmentation du pH du flux de butynediol brut et formation d'un flux de produit traité ;
(c) écoulement dudit flux de produit traité ayant un pH augmenté dans l'entrée d'une colonne de distillation continue ;
(d) récupération d'un flux de formaldéhyde concentré à partir du flux de distillat de tête de la colonne de distillation ; et
(e) récupération d'un flux de butynediol concentré à partir du flux de résidu de la colonne de distillation.

2. Procédé de la revendication 1 dans lequel le pH du flux de produit traité est augmenté à un niveau qui limite la réaction de butynediol et de formaldéhyde pour former un complexe acétal et diminuant la solubilité d'éléments traces métalliques présents dans le flux de produit traité, dans lequel les éléments traces métalliques précipitent dans le flux de produit traité.

3. Procédé de la revendication 1 dans lequel le niveau de pH du flux de produit traité est augmenté dans une plage de 5,5 à 10.

4. Procédé de la revendication 1 dans lequel le niveau de pH du flux de produit traité est augmenté par ajout d'un agent de contrôle du pH.

5. Procédé de la revendication 4 dans lequel l'agent de contrôle du pH est une solution aqueuse d'hydroxyde de sodium.

6. Procédé de la revendication 1 dans lequel le pH du flux de produit traité est augmenté au moyen de l'utilisation de résines d'échange d'ions.

7. Procédé de la revendication 1 dans lequel la concentration de formaldéhyde dans le flux de résidu de distillation est de 1,0 % en poids ou moins.

8. Procédé de la revendication 7 dans lequel la concentration de formaldéhyde dans le flux de résidu de distillation est de 0,1 à 1,0 % en poids.

9. Procédé de la revendication 1 comprenant en outre l'étape d'écoulement dudit flux de produit traité à travers un système de filtration pour éliminer les éléments traces métalliques avant l'étape (c).
